# EUROPEAN PATENT SPECIFICATION

(11) **EP 0 494 771 B1**
(45) Date of publication and mention of the grant of the patent: **05.04.1995**
(21) Application number: 92300153.1
(22) Date of filing: 08.01.1992
(51) Int. Cl.: C07D 309/20, C07D 309/22

(54) **Process for producing optically active dihydropyran derivatives**
Verfahren zur Herstellung optisch aktiver Dihydropyranderivate
Procédé pour la fabrication de dérivés de dihydropyranes optiquement actifs

(30) Priority: 08.01.1991 JP 11580/91
(43) Date of publication of application: 15.07.1992
(73) Proprietor: Takasago International Corporation, Tokyo 108 (JP)
(72) Inventor: Mikami, Koichi, Naka-ku, Yokohama-shi, Kanagawa (JP); Terada, Masahiro, Machida-shi, Tokyo (JP); Nakai, Takeshi, Yokohama-shi, Kanagawa (JP); Sayo, Noboru, Yokohama-shi, Kanagawa (JP)
(74) Representative: Moore, Anthony John

(56) References cited:
- CHEMICAL ABSTRACTS, vol. 90, no. 15, April 9, 1979, Columbus, Ohio, USA A.KONOWAL "2-Alkoxy-6-ace- thyl-5,6-dihydro-2H-pyrans" page 628, column 2,abstract-no. 121 419k
- CHEMICAL ABSTRACT, vol. 88, no. 21, May 22, 1978, Columnbus, Ohio, USA A.KONAWAL et al. "2-Alkoxy- 6-propionyl-5,6-dihydro-2H- pyrans" page 591, column1, abstract- no. 152 427z
- CHEMICAL ABSTRACTA, vol. 87, no. 21, November 1977, Columbus, Ohio, USA
- HOFFMANN-LA ROCHE "Polyenes" page 597, colums 2, abstract- no. 168 230v

## Description

The present invention relates to a process for producing optically active dihydropyran derivatives represented by formula (1): 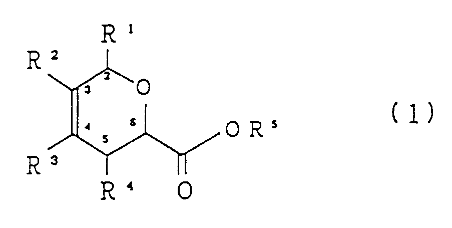
wherein R¹ and R⁴ each represents a lower alkyl group or a lower alkoxy group; R² and R³ each represents a hydrogen atom, a lower alkyl group, a lower alkoxy group; and R⁵ represents a lower alkyl group,
the optically active dihydropyran derivative having three optically active sites at the 2-, 5-, and 6- positions thereof, respectively.

Optically active dihydropyran derivatives represented by formula (1) are useful compounds, for example, as intermediates for syntheses of the saccharides described in A. KONOWA et al., Tetrahedron, Vol. 32, pp. 2957-2959 (1976) or of the antibiotics described in K.C. Nicolaou et al., J. Org. Chem., Vol. 50, p. 1440 (1985).

Hitherto, as processes for producing such optically active dihydropyran derivatives of formula (1), a process in which 1-methoxy-1,3-butadiene or 1,3-pentadiene is reacted with a glyoxylic acid ester in the presence of a catalyst which is menthoxyaluminum dichloride or Eu(hfc)₃, i.e., europium(III) tris[3-heptafluoropropylhydroxymethylene)-(⁺)-camphorate] has been reported in M. Quimpère et al., J. Chem. Soc., Chem. Commun., pp. 676-677 (1987).

However, the optically active site in the catalyst to be used in the process proposed by M. Quimpère et al. has a specific absolute configuration derived from a naturally occurring raw material, that is, absolute configurations for menthoxyaluminum dichloride and Eu(hfc)₃ are derived from (-)-menthol and (⁺)-camphor, respectively. However, even when products respectively having the absolute configurations corresponding to those of the two catalysts are intended to be obtained, the menthoxyaluminum dichloride catalyst cannot yield a desired product having an industrially utilizable optical purity. Hence, it has virtually been possible to produce only products having a specific absolute configuration obtained from the Eu(hfc)₃ catalyst derived from (⁺)-camphor. In addition, even with the Eu(hfc)₃ catalyst, attainable optical purities of the products are 64 %ee at maximum, which value is attained with (2R,6S)-2-methoxy-6-methoxycarbonyldihydropyran. It has, therefore, been desired to develop a process for producing a dihydropyran derivative having an even higher optical purity.

Some of the present inventors reported a process for producing a dihydropyran derivative having optically active sites at the 1- and 2-positions thereof in Lecture Preprint II of The 59Th Annual Meeting of The Chemical Society of Japan, p. 1356, Abstract No. 4D213, issued March 14, 1990. However, a process for producing a dihydropyran derivative wherein three optically active sites are determined has not yet been reported.

Under these circumstances, the present inventors have conducted intensive studies in order to overcome the above-described problems. As a result, it has now been found that when an optically active binaphthol-titanium complex is used as a catalyst, a dihydropyran derivative with a high optical purity, which has three optically active sites in the molecule thereof, can be obtained efficiently. The present invention has been completed based on this finding.

Accordingly, an object of the present invention is to provide a process for producing a dihydropyran derivative represented by formula (1) given above.

Other objects and effects of the present invention will be apparent from the following description.

The process of the present invention is illustrated by the following reaction scheme: 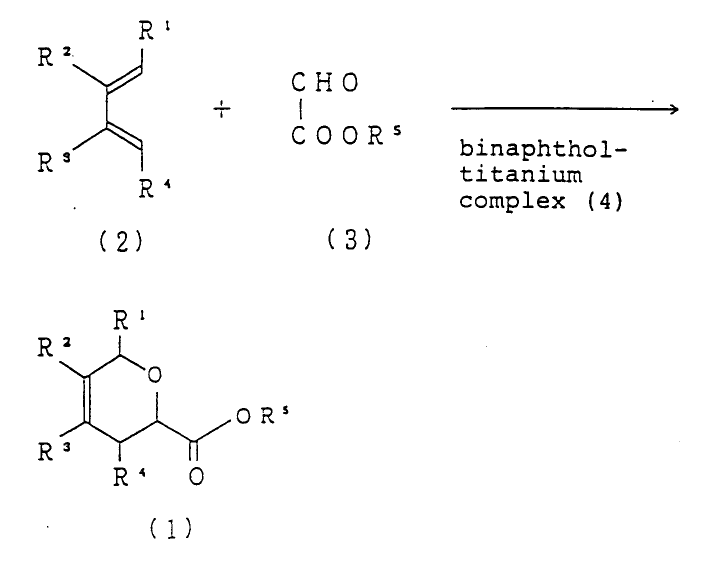
wherein R¹, R², R³, R⁴, and R⁵ are as defined hereinabove.

That is, in the process of the present invention, a diene compound (2) is reacted with a glyoxylic acid ester (3) in the presence of an optically active binaphthol-titanium complex (4) to produce an optically active dihydropyran derivative (1).

The alkyl moiety in the lower alkyl group or lower alkoxy group as referred to herein preferably has from 1 to 4 carbon atoms.

Examples of the diene compound (2) include 2,4-hexadiene, 2,4-heptadiene, 3-methyl-2,4-hexadiene, 3,4-dimethyl-2,4-hexadiene, 1-methoxy-1,3-pentadiene, 1-methoxy-2-methyl-1,3-pentadiene, 1-methoxy-2,3-dimethyl-1,3-pentadiene, 3-ethoxy-2,4-hexadiene, 3-t-butoxy-2,4-hexadiene,and 3-ethoxy-4-methyl-2,4-hexadiene.

Examples of the glyoxylic acid ester (3) which is another raw material include methyl glyoxylate, ethyl glyoxylate, isopropyl glyoxylate, and t-butyl glyoxylate. These glyoxylic acid esters may be produced, for example, by the method proposed by T. ROSS KELLY et al., Synthesis, pp. 544-545 (1972).

The optically active binaphthol-titanium complex used as a catalyst is represented by formula (4): 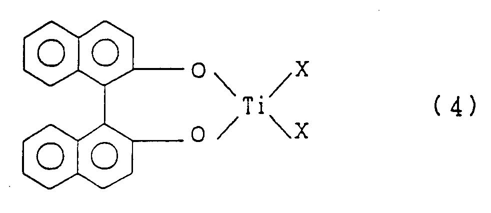
wherein X represents a chlorine atom or a bromine atom.

This binaphthol-titanium complex (4) can be prepared, for example, by the method described in JP-A-2-40344 (corresponding to European Patent 353,053A). (The term "JP-A" as used herein means an "unexamined published Japanese patent application".) That is, a titanium tetrahalide (the halogen being chlorine or bromine) and tetraisopropoxytitanium are first mixed with each other in hexane to form crystals of a diisopropoxydihalogenotitanium, which are then dissolved in toluene. Meanwhile, a powder of molecular sieves 4A (a product on the market) is added to methylene chloride in an amount of at least 0.5 g per mmole of the catalyst. To this mixture are added the aboveprepared diisopropoxydihalogenotitanium toluene solution and then binaphthol. The resulting mixture is stirred for about 1 hour, thereby giving the binaphthol-titanium complex (4).

The binaphthol-titanium complex (4) includes an (R)-isomer and an (S)-isomer which are synthesized from (R)-binaphthol and (S)-binaphthol, respectively. These isomers can be suitably selected and used according to the desired absolute configuration for the optically active dihydropyran derivative (1) to be produced. Illustratively stated, in the case where the dihydropyran derivative to be obtained is the (R)-isomer with respect to the asymmetric carbon at the 6-position on the dihydropyran ring in formula (1), (R)-(4) can be used; in the case where the (S)-isomer is to be obtained, (S)-(4) can be used. Thus, according to the present invention, the absolute configuration of the carbon atom at the 6-position can be freely determined by suitably selecting the complex (4) to be used. Further, the carbon atoms at the 2- and 5-positions of the compound of formula (1) are also asymmetric. In this case, however, either the (R)-isomer or the (S)-isomer can be obtained in an advantageous proportion according to the absolute configuration for the complex (4).

In practicing the process of the present invention, the diene compound (2) and the glyoxylic acid ester (3) are added to a solution of the binaphthol-titanium complex (4) in an organic solvent, and then the mixture is allowed to react.

Examples of the organic solvent that can be used in the present invention include halogenated hydrocarbons such as methylene chloride, chloroform, and carbon tetrachloride; aromatic hydrocarbons such as benzene and toluene; and aprotic solvents such as tetrahydrofuran, diethyl ether, and dimethoxyethane.

The amount of the binaphthol-titanium complex (4) used as a catalyst is generally in the range of from 0.01 to 1 mole, and preferably from 0.05 to 0.1 mole, per mole of the raw materials (2) and (3), from the standpoint of obtaining the desired product in a high optical yield. The reaction temperature is generally in the range of from -50 to 0°C, and preferably from -30 to -10°C. The reaction time is preferably from 1 to 20 hours.

After the reaction, an alkaline agent, e.g., a sodium hydrogencarbonate aqueous solution, is added to the reaction mixture. Subsequently, the resulting mixture is subjected to extraction with a solvent such as diethyl ether and ethyl acetate. After drying, the solvent is removed by evaporation, and the residue is purified by column chromatography with silica gel, etc., whereby the desired, optically active dihydropyran derivative (1) can be obtained in a high yield.

The process of the present invention can be, for example, applied to a process for producing an optically active dihydropyran derivative having a fused ring, such as production of methyl 3,6,7,8a-tetrahydro-2H,5H-pyrano[2,3-b]pyran-2-carboxylate (7) from 3-ethylene-4,5-dihydropyran (5) and methyl glyoxylate (6) as illustrated in the following reaction scheme. 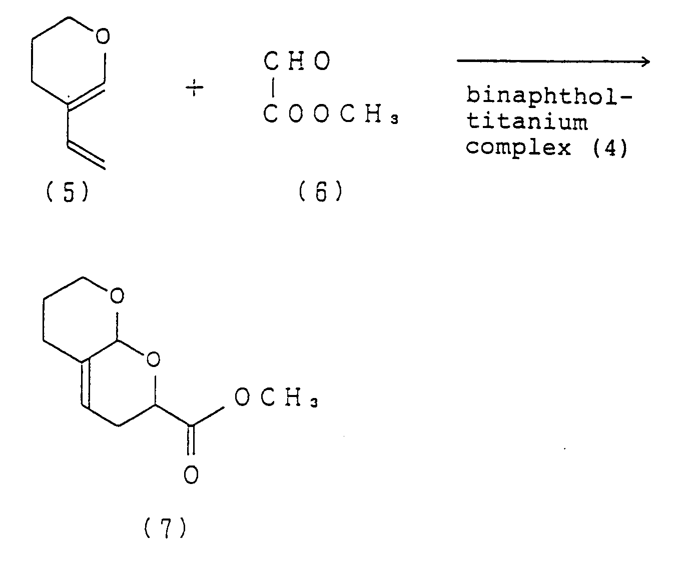
As described above, according to the process of the present invention, optically active dihydropyran derivatives with high optical purities can be produced from diene compounds and glyoxylic acid esters by use of an optically active binaphthol-titanium complex as a catalyst. Therefore, the process of the present invention is industrially advantageous.

The present invention will be explained in more detail with reference to the following Examples, which should not be construed to be limiting the scope of the invention.

In the Examples, the following analytical instruments were used for respective analyses.
¹H Nuclear Magnetic Resonance Spectroscopy (hereinafter abbreviated as ¹H-NMR):
Type GEMINI 200 (200 MH_{z}) (manufactured by Varian Co.) Measurement of Optical Rotation:
Polarimeter DIP-370 (manufactured by JASCO Ltd.)

### EXAMPLE 1

Into a 50-ml Schlenk's tube which had been displaced by argon beforehand were introduced 2.98 ml (10 mmole) of tetraisopropoxytitanium and 5 ml of hexane and then 1.10 ml (10 mmole) of titanium tetrachloride. The mixture was stirred at room temperature for 10 minutes and then allowed to stand at room temperature for 3 hours, upon which white crystals precipitated. The solvent was taken out with a syringe, and 5 ml of hexane was added to the residue to recrystallize it. This procedure was repeated twice, and the resulting crystals were then dried under reduced pressure, thereby obtaining 3.09 g of white diisopropoxydichlorotitanium. 43 ml of toluene was added thereto to prepare a 0.3N solution.

On the other hand, 0.5 g of a powder of molecular sieves 4A (manufactured by Aldrich Co.) was placed in a 25-ml flask, and the air in the flask was thoroughly displaced by argon. 5 ml of methylene chloride was added thereto, and 0.33 ml (0.1 mmole) of the above-prepared diisopropoxydichlorotitanium toluene solution and 28.6 mg (0.1 mmole) of (R)-binaphthol were further added. The mixture was stirred at room temperature for 1 hour, thereby preparing an (R)-binaphtholdichlorotitanium complex.

The above-obtained solution was cooled to -70°C in a dry ice-acetone bath. To this solution were successively added 88 mg (1 mmole) of methyl glyoxylate and 0.196 g (2 mmole) of 1-methoxy-1,3-pentadiene. Reaction was then allowed to proceed at -10°C for 1 hour, and 10 ml of a sodium hydrogencarbonate aqueous solution was added to the reaction mixture to terminate the reaction. This reaction mixture was filtered through Celite and then subjected to extraction once with a 20 ml of diethyl ether and twice with 20 ml of ethyl acetate. The extract was dried over anhydrous magnesium sulfate. Thereafter, the solvent was removed by evaporation, and the residue was purified by silica gel column chromatography (200 mesh; developing solvent: hexane/ethyl acetate = 10/1), thereby obtaining 0.23 g of desired optically active 2-methoxy-5-methyl-6-methoxycarbonyl-5,6-dihydropyran(percent yield:63%).

The ¹H-NMR analysis revealed that the ratio of the endo-isomer to the exo-isomer yielded was 97:3.
¹H-NMR (CDCl₃) δppm:
- Endo-isomer (2S,5S,6R):: 1.04 (d, J=6.8Hz, 3H), 2.25 (m, 1H), 3.53 (s, 3H), 3.79 (s, 3H), 4.43 (d, J=3.6Hz, 1H), 5.16 (bs, 1H), 5.62 (m, 1H), 6.02 (m, 1H)
- Exo-isomer (2R,5R,6R):: 1.05 (d, J=7.2Hz, 3H), 2.55 (m, 1H), 3.45 (s, 3H), 3.82 (s, 3H), 4.09 (d, J=10.4Hz, 1H), 4.95 (m, 1H), 5.73 (m, 1H), 5.81 (m, 1H)

The optical purity of the product was determined by ¹H-NMR analysis using an optically active shifting agent, (⁺)-Eu(DPPM)₃ [(⁺)-europium(III) tris[di(perfluoro-2-propoxypropionyl)methanate], manufactured by Daiichi Pure Chemicals Co., Ltd.]. As a result, it was found that the optical purity of the endo-isomer (2S,5S,6R) was 90 %ee. On the other hand, the yield of the exo-isomer was very low so that the optical purity thereof was unmeasurable.
Optical rotation:
[α]_{D}³⁰ = +163° (c=0.285, chloroform)

### EXAMPLE 2

In the same manner as in Example 1, a solution of an (R)-binaphthol-dichlorotitanium complex was obtained. This solution was cooled to -70°C in a dry ice-acetone bath. To this solution were successively added 88 mg (1 mmole) of methyl glyoxylate and 224 mg (2 mmole) of 1-methoxy-2-methy-1,3-pentadiene. Reaction was then allowed to proceed at -30°C for 1 hour, and 10 ml of a sodium hydrogencarbonate aqueous solution was added to the reaction mixture to terminate the reaction. This reaction mixture was filtered through Celite and then subjected to extraction once with 20 ml of diethyl ether and twice with 20 ml of ethyl acetate. The extract was dried over anhydrous magnesium sulfate. Thereafter, the solvents were removed by evaporation, and the residue was purified by silica gel column chromatography (200 mesh; developing solvent: hexane/ethyl acetate = 10/1), thereby obtaining 108 mg of desired optically active 2-methoxy-3,5-dimethyl-6-methoxycarbonyl-5,6-dihydropyran (percent yield: 54%).

The ¹H-NMR analysis revealed that the ratio of the endo-isomer to the exo-isomer yielded was 98:2.
¹H-NMR (CDCl₃) δppm:
- Endo-isomer (2S,5S,6R):: 1.01 (d, J=7.0Hz, 3H), 1.66 (m, 3H), 2.46 (m, 1H), 3.48 (s, 3H), 3.78 (s, 3H), 4.38 (d, J=3.5Hz, 1H), 5.04 (m, 1H), 5.73 (m, 1H)
- Exo-isomer (2R,5R,6R):: 1.02 (d, J=7.1Hz, 3H), 1.72 (m, 3H), 2.51 (m, 1H), 3.46 (s, 3H), 3.81 (s, 3H), 4.03 (d, J=10.5Hz, 1H), 4.73 (m, 1H), 5.43 (m, 1H)

Optical purity:
Endo-isomer (2S,5S,6R): 88 %ee
Exo-isomer: unmeasurable due to very low yield

### REFERENCE EXAMPLE

In the same manner as in Example 1, a solution of an (R)-binaphthol-dichlorotitanium complex was obtained. This solution was cooled to -70°C in a dry ice-acetone bath. To this solution were succesively added 88 mg (1 mmole) of methyl glyoxylate and 220 mg (2 mmole) of 3-ethylene-4,5-dihydropyran. Reaction was then allowed to proceed at -30°C for 3 hours, and 10 ml of a sodium hydrogencarbonate aqueous solution was added to the reaction mixture to terminate the reaction. This reaction mixture was filtered through Celite and then subjected to extraction once with 20 ml of diethyl ether and twice with 20 ml of ethyl acetate. The extract was dried over anhydrous magnesium sulfate. Thereafter, the solvent was removed by evaporation, and the residue was purified by silica gel column chromatography (200 mesh; developing solvent: hexane/ethyl acetate = 10/1), thereby obtaining 123 mg of desired optically active methyl 3,6,7,8a-tetrahydro-2H,5H-pyrano[2,3-b]pyran-2-carboxylate (percent yield: 62%).

The ¹H-NMR analysis revealed that the ratio of the cis-isomer to the trans-isomer yielded was 89:11.
¹H-NMR (CDCl₃) δppm:
- Cis-isomer (2R,8aS):: 1.68 (m, 2H), 2.33 (m, 4H), 3.63 (m, 1H), 3.78 (s, 3H), 4.06 (m, 1H), 4.38 (dd, J=4.4Hz, J=9.2Hz, 1H), 5.14 (br, s, 1H), 5.64 (m, 1H)
- Trans-isomer (2R,8aR):: 1.68 (m, 2H), 2.33 (m, 4H), 3.65 (m, 1H), 3.79 (s, 3H), 4.06 (m, 1H), 4.47 (dd, J=7.0Hz, J=8.2Hz, 1H), 4.98 (br, s, 1H), 5.64 (m, 1H)

Optical purity:
Cis-isomer (2R,8aS): 86 %ee
Trans-isomer: unmeasurable due to very low yield

## Claims

1. A process for producing an optically active dihydropyran derivative represented by formula (1): 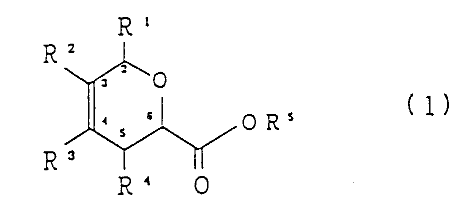 wherein R¹ and R⁴ each represents a lower alkyl group or a lower alkoxy group; R² and R³ each represents a hydrogen atom, a lower alkyl group, a lower alkoxy group; and R⁵ represents a lower alkyl group,
which comprises reacting a diene compound represented by formula (2): 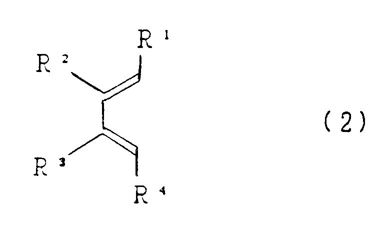 wherein R¹, R², R³, and R⁴ have the same meanings as defined above,
with a glyoxylic acid ester represented by formula (3): 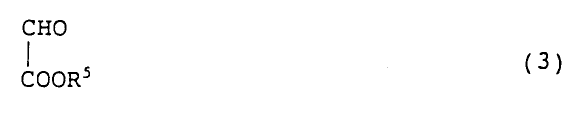 wherein R⁵ has the same meaning as defined above,
in the presence of an optically active binaphthol-titanium complex represented by formula (4): 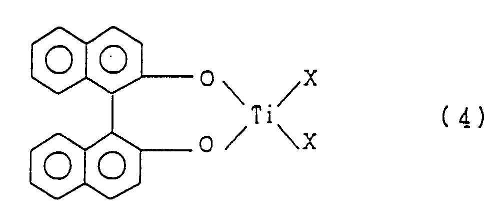 wherein X represents a chlorine atom or a bromine atom.

2. A process as in claim 1, wherein said binaphthol-titanium complex represented by formula (4) is used in an amount of from 0.01 to 1 mole per mole of said diene compound represented by formula (2) and said glyoxylic acid ester represented by formula (3).

3. A process as in claim 2, wherein said binaphthol-titanium complex represented by formula (4) is used in an amount of from 0.05 to 0.1 mole per mole of said diene compound represented by formula (2) and said glyoxylic acid ester represented by formula (3).

4. A process as claimed in claim 1, 2 or 3, wherein the alkyl moiety in the lower alkyl groups or lower alkoxy groups has from 1 to 4 carbon atoms.

## Patentansprüche

1. Verfahren zum Herstellen eines optisch aktiven Dihydropyranderivats, das durch die Formel (1) dargestellt ist: 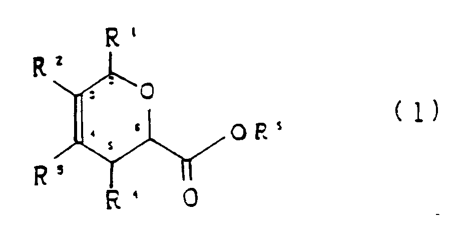 worin R¹ und R⁴ jeweils eine niedere Alkylgruppe oder eine niedere Alkoxygruppe darstellt; R² und R³ jeweils ein Wasserstoffatom, eine niedere Alkylgruppe, eine niedere Alkoxygruppe darstellt; und R⁵ eine niedere Alkylgruppe darstellt,
welches das Umsetzen einer Dienverbindung, die durch Formel (2) dargestellt ist: 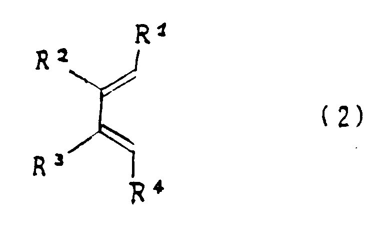 worin R¹, R², R³ und R⁴ die gleichen Bedeutungen wie oben definiert haben,
mit einem Glyoxylsäureester, der durch Formel (3) dargestellt ist: 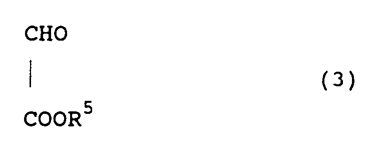 worin R⁵ die gleiche Bedeutung wie oben definiert hat,
in Gegenwart eines optisch aktiven Binaphthol-Titan-Komplexes, der durch Formel (4) dargestellt ist, umfaßt: 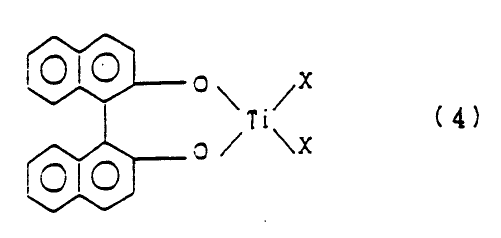 worin X ein Chloratom oder ein Bromatom bedeutet.

2. Verfahren nach Anspruch 1, worin der durch Formel (4) dargestellte Binaphthol-Titan-Komplex in einer Menge von 0,01 bis 1 Mol pro Mol der durch Formel (2) dargestellten Dienverbindung und des durch Formel (3) dargestellten Glyoxylsäureesters verwendet wird.

3. Verfahren nach Anspruch 2, worin der durch Formel (4) dargestellte Binaphthol-Titan-Komplex in einer Menge von 0,05 bis 0,1 Mol pro Mol der durch Formel (2) dargestellten Dienverbindung und des durch Formel (3) dargestellten Glyoxylsäureesters verwendet wird.

4. Verfahren nach Anspruch 1, 2 oder 3, worin der Alkylbestandteil in den niederen Alkylgruppen oder niederen Alkoxygruppen 1 bis 4 Kohlenstoffatome hat.

## Revendications

1. Procédé pour fabriquer un dérivé de dihydropyranne optiquement actif représenté par la formule (1): 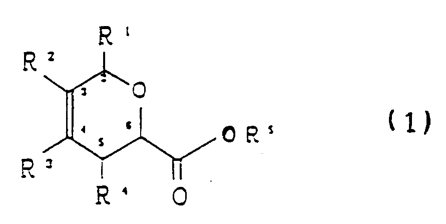 dans laquelle R¹ et R⁴ représentent chacun un groupe alkyle inférieur ou un groupe alcoxy inférieur; R² et R³ représentent chacun un atome d'hydrogène, un groupe alkyle inférieur, un groupe alcoxy inférieur; et R⁵ représente un groupe alkyle inférieur,
qui comprend la réaction d'un diène représenté par la formule (2): 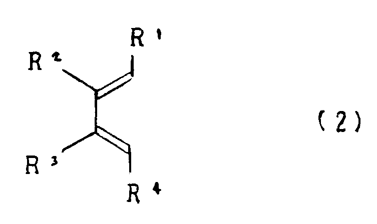 dans laquelle R¹, R², R³ et R⁴ sont définis comme ci-dessus,
avec un ester d'acide glyoxylique représenté par la formule (3): 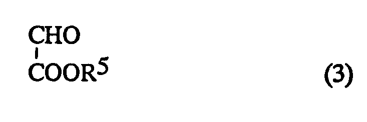 dans laquelle R⁵ est défini comme ci-dessus,
en présence d'un complexe binaphtol-titane optiquement actif représenté par la formule (4): 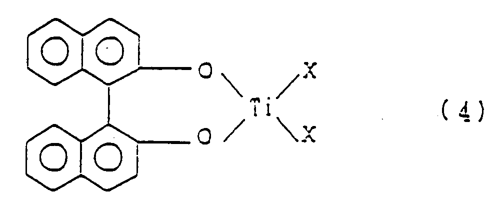 dans laquelle X représente un atome de chlore ou un atome de brome.

2. Procédé selon la revendication 1, dans lequel ledit complexe binaphtol-titane représenté par la formule (4) est utilisé en quantité de 0,01 à 1 mol par mole dudit diène représenté par la formule (2) et dudit ester d'acide glyoxylique représenté par la formule (3).

3. Procédé selon la revendication 2, dans lequel ledit complexe binaphtol-titane représenté par la formule (4) est utilisé en quantité de 0,05 à 0,1 mole par mole dudit diène représenté par la formule (2) et dudit ester d'acide glyoxylique représenté par la formule (3).

4. Procédé selon la revendication 1, 2 ou 3, dans lequel le reste alkyle dans les groupes alkyles inférieurs ou les groupes alcoxy inférieurs a de 1 à 4 atomes de carbone.
